# EUROPEAN PATENT APPLICATION

(11) **EP 4 501 223 A1**
(43) Date of publication of application: **05.02.2025**
(21) Application number: 23910259.3
(22) Date of filing: 19.12.2023
(51) Int. Cl.: A61B 5/024, A61B 5/1455

(54) **MULTI-LIGHT-SOURCE HEART RATE MEASUREMENT APPARATUS AND METHOD, AND WEARABLE DEVICE**

(30) Priority: 30.12.2022 CN 202211726789
(71) Applicant: Kingfar International Inc., 100085 Beijing (CN)
(72) Inventor: ZHAO, Qichao, Beijing 100085 (CN); YANG, Ran, Beijing 100085 (CN)
(74) Representative: Dr. Gassner & Partner mbB
(86) International application number: PCT/CN2023/139828
(87) International publication number: WO 2024/140329

(57) **Abstract**

Provided in the present application are a multi-light-source heart rate measurement apparatus and method, and a wearable device. The multi-light-source heart rate measurement apparatus comprises: a first optical receiving group, a second optical receiving group, a first light source, and a second light source, wherein the distance between the first light source and the first optical receiving group and the distance between the first light source and the second optical receiving group are both preset resting and first dynamic heart rate measurement intervals; the distance between the second light source and the first optical receiving group is a preset resting heart rate measurement interval, and the resting heart rate measurement interval is less than or equal to the resting and first dynamic heart rate measurement interval, and the distance between the second light source and the second optical receiving group is a preset second dynamic heart rate measurement interval, and the second dynamic heart rate measurement interval is greater than the resting and first dynamic heart rate measurement interval.

## Description

### TECHNICAL FIELD

The present application relates to a field of wearable devices, and more particularly to a multi-light source heart rate measurement apparatus, a method and a wearable device.

### BACKGROUND ART

A wearable device can measure physiological parameter information such as heart rate and oxygen saturation by setting a Photo Plethysmo Graphy (PPG) sensor and based on a PPG technology. PPG detection technology is mainly divided into two types from a perspective of PPG sensor layout: one is a transmission detection technique and a reflection detection technique, in which PPG sensors used on wearable devices typically measure physiological parameter information at a wrist using the reflection detection technique. Currently, PPG is mainly applied to a smart watch or a bracelet for resting heart rate measurement and exercise heart rate measurement.

The existing wearable PPG sensor device uses two photoplethysmography pulse wave sensors distributed at different positions to collect photoplethysmography pulse wave signals of two channels, and simultaneously uses a three-axis accelerometer to collect a motion acceleration signal in the same time period. A sampled signal needs to be filtered by a second order Butterworth filter with a pass band of 0.4 Hz-4 Hz to eliminate an interference of motion noise and other noise outside a certain frequency range. Frequency positions of motion noise in two spectrums of the photoplethysmography pulse wave signals are aligned with the frequency positions of the spectrum of the motion acceleration signal, and two spectrums of the photoplethysmography pulse wave signals with motion noise removed, i.e., a clean PPG signal spectrum, can be obtained by spectral subtraction.

There are only two optical paths in the prior art, and it is necessary to implement either two optical paths with the same distance between PD and LED or two optical paths with different distances together with an accelerometer, and it is impossible to implement heart rate measurement for multiple scenes of resting, slight exercise and vigorous exercise without using the accelerometer to participate in a calculation of PPG.

### SUMMARY

In view of the above, embodiments of the present application provide a multi-light source heart rate measurement apparatus, a method and a wearable device to obviate or ameliorate one or more disadvantages of the prior art.

In a first aspect, the present application provides a multi-light source heart rate measurement apparatus, including:
a first optical receiving group, a second optical receiving group, a first light source and a second light source;
wherein distances between the first light source and the first optical receiving group and the second optical receiving group respectively are both a preset resting and first dynamic heart rate measurement interval, based on the first light source cooperating with the first optical receiving group and/or the second optical receiving group, a resting heart rate of a person to be tested in a non-exercise state or a first dynamic heart rate of the person to be tested in a first exercise state is measured;
the distance between the second light source and the first optical receiving group is a preset resting heart rate measurement interval, and the resting heart rate measurement interval is less than or equal to the resting and first dynamic heart rate measurement interval; the resting heart rate of the person to be tested is measured based on the second light source cooperating with the first optical receiving group;
the distance between the second light source and the second optical receiving group is a preset second dynamic heart rate measurement interval, and the second dynamic heart rate measurement interval is larger than the resting and first dynamic heart rate measurement interval, a second dynamic heart rate of the person to be tested in a second exercise state is measured based on the second light source cooperating with the second optical receiving group, wherein an intensity of exercise of the second exercise state is greater than that of the first exercise state.

In a second aspect, the present application provides a wearable device including:
a nine-axis sensor and the multi-light source heart rate measurement apparatus as described in the preceding embodiments;
the multi-light source heart rate measurement apparatus is arranged on a housing of the wearable device for contacting a skin of the person to be tested; the first optical receiving group, the second optical receiving group, the first light source and the second light source are respectively in communication connection with a processor in the wearable device;
the nine-axis sensor is used for monitoring an acceleration data of the person to be tested in real time, and sending the acceleration data to the processor in the wearable device in real time, so that the processor determines a current intensity of exercise of the person to be tested based on the acceleration data, and controls at least one of the first light source and the second light source to be turned on or off according to the intensity of exercise, so that an optical signal reflected by blood and tissues of the person to be tested is received by the first optical receiving group and/or the second optical receiving group, and the optical signal is converted into an electrical signal to be sent to an analog-to-digital conversion chip; the analog-to-digital conversion chip converts the electrical signal into a digital signal and sends the digital signal to the processor, so that the processor determines a corresponding pulse signal based on a digital information, and then determines a heart rate corresponding to the person to be tested based on the pulse signal.

In a third aspect, the present application provides a multi-light source heart rate detection method, which is implemented by using the multi-light source heart rate measurement apparatus described in the preceding embodiments;
the multi-light source heart rate detection method includes:
judging a current state of the person to be tested based on an acceleration data of the person to be tested collected in real time by a nine-axis sensor, wherein the state comprises: the non-exercise state, the first exercise state and the second exercise state, and the intensity of exercise of the second exercise state is higher than that of the first exercise state; and
applying the multi-light source heart rate measurement apparatus to acquire a corresponding pulse signal according to the current exercise state of the person to be tested to determine a heart rate corresponding to the person to be tested based on the pulse signal.

The present application provides a multi-light source heart rate measurement apparatus, a method and a wearable device, which can realize heart rate measurement under multiple scenes of resting, slight exercise, and vigorous exercise without using the accelerometer to participate in the calculation of PPG by setting the intervals between different light sources and different optical receiving groups.

Additional advantages, objects, and features of the present invention will be set forth in part in the description which follows and in part will become apparent to a person skilled in the art upon examination of the following or may be learned from practice of the present application. The objects and other advantages of the present application may be realized and attained by a structure particularly pointed out in the description and drawings.

### BRIEF DESCRIPTION OF THE DRAWINGS

FIG. 1 is a schematic view of a structure of a multi-light source heart rate measurement apparatus in an embodiment of the present application.
FIG. 2 is a flowchart of a multi-light source heart rate detection method in another embodiment of the present application.

### DETAILED DESCRIPTION

The present application will be described in further detail with reference to the following embodiments and drawings, in order to make the objects, technical solutions and advantages of the present application more apparent. The illustrative embodiments and description herein are to be considered in all respects as illustrative and not restrictive.

It should be emphasized that a term "include/including" when used herein is taken to specify a presence of stated features, elements, steps or components, but does not preclude the presence or addition of one or more other features, elements, steps or components.

It is also noted herein that, unless otherwise specified, the term "connect/connection" may refer herein not only to a direct connection, but also to an indirect connection where an intermediate is present.

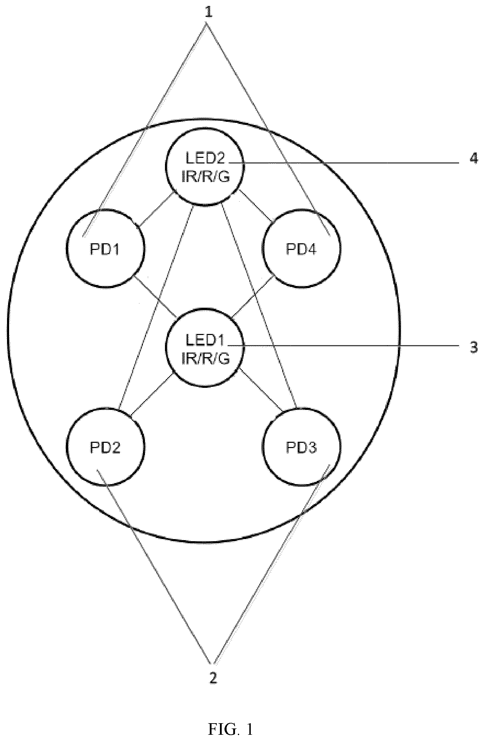

Embodiments of the present application provide a multi-light source heart rate measurement apparatus. With reference to FIG. 1, the multi-light source heart rate measurement apparatus specifically includes the following contents:
a first optical receiving group 1, a second optical receiving group 2, a first light source 3 and a second light source 4;
wherein distances between the first light source 3 and the first optical receiving group 1 and the second optical receiving group 2 respectively are both a preset resting and first dynamic heart rate measurement interval, based on the first light source 3 cooperating with the first optical receiving group 1 and/or the second optical receiving group 2, a resting heart rate of a person to be tested in a non-exercise state or a first dynamic heart rate of the person to be tested in a first exercise state is measured;
the distance between the second light source 4 and the first optical receiving group 1 is a preset resting heart rate measurement interval, and the resting heart rate measurement interval is less than or equal to the resting and first dynamic heart rate measurement interval; the resting heart rate of the person to be tested is measured based on the second light source 4 cooperating with the first optical receiving group 1;
the distance between the second light source 4 and the second optical receiving group 2 is a preset second dynamic heart rate measurement interval, and the second dynamic heart rate measurement interval is larger than the resting and first dynamic heart rate measurement interval, a second dynamic heart rate of the person to be tested in a second exercise state is measured based on the second light source 4 cooperating with the second optical receiving group 2, wherein an intensity of exercise of the second exercise state is greater than that of the first exercise state.

Specifically, the first optical receiving group 1 corresponds to PD1 and PD4, the second optical receiving group 2 corresponds to PD2 and PD3, the first light source 3 corresponds to LED1, the second light source 4 corresponds to LED2, and distances between the first light source 3 and the first optical receiving group 1 and the second optical receiving group 2 respectively are both the preset resting and first dynamic heart rate measurement interval, based on the first light source 3 cooperating with the first optical receiving group 1 and/or the second optical receiving group 2, the resting heart rate of the person to be tested in the non-exercise state or the first dynamic heart rate of the person to be tested in the first exercise state is measured; the distance between the second light source 4 and the first optical receiving group 1 is the preset resting heart rate measurement interval, and the resting heart rate measurement interval is less than or equal to the resting and first dynamic heart rate measurement interval, the resting heart rate of the person to be tested is measured based on the second light source 4 cooperating with the first optical receiving group 1; the distance between the second light source 4 and the second optical receiving group 2 is a preset second dynamic heart rate measurement interval, and the second dynamic heart rate measurement interval is larger than the resting and first dynamic heart rate measurement interval, a second dynamic heart rate of the person to be tested in the second exercise state is meatured based on the second light source 4 and the second optical receiving group 2, wherein the intensity of exercise of the second exercise state is greater than that of the first exercise state. Heart rate measurement under multiple scenes of resting, slight exercise, and vigorous exercise can be achieved without using the accelerometer to participate in the calculation of PPG by setting the intervals between different light sources and different optical receiving groups.

In some embodiments of the present application, the first light source 3 and the second light source 4 are respectively in communication connection with a processor of a wearable device such that the processor controls at least one of the first light source 3 and the second light source 4 to be turned on or off.

Specifically, the processor of the wearable device is in communication connection with the first light source 3 and the second light source 4, respectively, and controlls at least one of the first light source 3 and the second light source 4 to be turned on or off for independently controlling the first light source 3 and the second light source 4. The processor of the wearable device enables independent control of the light source, which improves an independence and stability of a heart rate measurement process.

In some embodiments of the present application, the first light source 3 and the second light source 4 are both light-emitting diodes for emitting red light, infrared light, and green light;
the first light source emits the green light when used to measure the first dynamic heart rate of the person to be tested in the first exercise state and the second light source emits the green light when used to measure the second dynamic heart rate of the person to be tested in the second exercise state.

Specifically, the first light source 3 and the second light source 4 are both light-emitting diodes for emitting red light, infrared light, and green light; wherein a peak wavelength of red light is 660 nm, the peak wavelength of infrared light is 950 nm, and the peak wavelength of green light is 526 nm; the first light source 3 emits green light when used to measure the first dynamic heart rate of the person to be tested in the first exercise state and the second light source 4 when used to measure the second dynamic heart rate of the person to be tested in the second exercise state. The light emitting diodes using a combination of red, infrared and green light can be used flexibly according to specific requirements of the heart rate measurement.

In some embodiments of the present application, both the first optical receiving group 1 and the second optical receiving group 2 include at least two optical receivers;
the distance between each of the optical receivers and the first light source 3 is the resting and first dynamic heart rate measurement interval;
the distances between the optical receivers in the first optical receiving group 1 and the second light source 4 are both the resting heart rate measurement interval; and
the distances between the optical receivers in the second optical receiving group 2 and the second light source 4 are both the second dynamic heart rate measurement interval.

Specifically, the first optical receiving group 1 and the second optical receiving group 2 each includes at least two optical receivers; the distance between each of the optical receivers and the first light source 3 is the resting and first dynamic heart rate measurement interval; the distances between the optical receivers in the first optical receiving group 1 and the second light source 4 are both the resting heart rate measurement interval; the distances between the optical receivers in the second optical receiving group 2 and the second light source 4 are both the second dynamic heart rate measurement interval. An accuracy of the heart rate measurement can be improved by arranging at least two optical receivers in each optical reception group.

In some embodiments of the present application, a value of the resting heart rate measurement interval ranges from 2 mm to 5 mm;
the value of the resting and first dynamic heart rate measurement interval ranges from 4 mm to 5 mm; and
the value of the second dynamic heart rate measurement interval ranges from 5 mm to 9 mm.

Specifically, the value of the resting heart rate measurement interval ranges from 2 mm to 5 mm; the value of the resting and first dynamic heart rate measurement interval ranges from 4 mm to 5 mm; and the value of the second dynamic heart rate measurement interval ranges from 5 mm to 9 mm. In the present application, 2 mm to 3 mm with the best heart rate measurement effect is selected in the range of the resting heart rate measurement interval, and 7 mm to 9 mm with the best heart rate measurement effect is selected in the range of the second dynamic heart rate measurement interval. The accuracy of the heart rate measurement can be improved by setting an optimal measurement interval.

The present application provides a wearable device, wherein the wearable device is provided with a nine-axis sensor and the multi-light source heart rate measurement apparatus as described in the preceding embodiments;
the multi-light source heart rate measurement apparatus is arranged on a housing of the wearable device for contacting a skin of the person to be tested; the first optical receiving group 1, the second optical receiving group 2, the first light source 3 and the second light source 4 are respectively in communication connection with a processor in the wearable device;
the nine-axis sensor is used for monitoring an acceleration data of the person to be tested in real time, and sending the acceleration data to the processor in the wearable device in real time, so that the processor determines a current intensity of exercise of the person to be tested based on the acceleration data, and controls at least one of the first light source 3 and the second light source 4 to be turned on or off according to the intensity of exercise, so that an optical signal reflected by blood and tissues of the person to be tested is received by the first optical receiving group 1 and/or the second optical receiving group 2, and the optical signal is converted into an electrical signal to be sent to an analog-to-digital conversion chip; the analog-to-digital conversion chip converts the electrical signal into a digital signal and sends the digital signal to the processor, so that the processor determines a corresponding pulse signal based on a digital information, and then determines a heart rate corresponding to the person to be tested based on the pulse signal.

Specifically, the wearable device is provided with the nine-axis sensor and the multi-light source heart rate measurement apparatus as described in the preceding embodiments; the multi-light source heart rate measurement apparatus is arranged on the housing of the wearable device for contacting the skin of the person to be tested; the first optical receiving group 1, the second optical receiving group 2, the first light source 3 and the second light source 4 are respectively in communication connection with the processor in the wearable device; the nine-axis sensor is used for monitoring the acceleration data of the person to be tested in real time, and sending the acceleration data to the processor in the wearable device in real time, so that the processor determines the current intensity of exercise of the person to be tested based on the acceleration data, and controls at least one of the first light source 3 and the second light source 4 to be turned on or off according to the intensity of exercise, so that the optical signal reflected by the blood and tissues of the person to be tested is received by the first optical receiving group 1 and/or the second optical receiving group 2, and the optical signal is converted into an electrical signal to be sent to the analog-to-digital conversion chip; the analog-to-digital conversion chip converts the electrical signal into a digital signal and sends the digital signal to the processor, so that the processor determines the corresponding pulse signal based on the digital information, and then determines the heart rate corresponding to the person to be tested based on the pulse signal. Through a collaborative use of nine-axis sensor and multi-light source heart rate measurement apparatus in the wearable device, the exercise state of the person to be tested is clarified, and the accuracy of the heart rate measurement is improved.

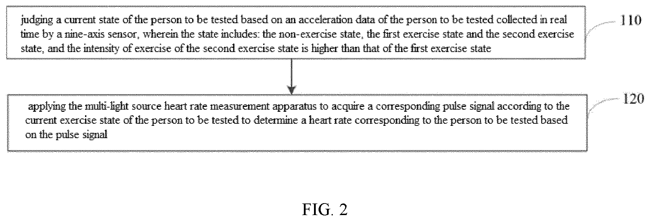

Embodiments of the present application also provide a multi-light source heart rate detection method implemented by the multi-light source heart rate measurement apparatus described in the preceding embodiments, as shown in FIG. 2;
the multi-light source heart rate detection method includes:
Step 110: judging a current state of the person to be tested based on an acceleration data of the person to be tested collected in real time by a nine-axis sensor, wherein the state includes: the non-exercise state, the first exercise state and the second exercise state, and the intensity of exercise of the second exercise state is higher than that of the first exercise state ; and
Step 120: applying the multi-light source heart rate measurement apparatus to acquire a corresponding pulse signal according to the current exercise state of the person to be tested to determine a heart rate corresponding to the person to be tested based on the pulse signal.

Specifically, the processor in the wearable device judges the current state of the person to be tested based on the acceleration data of the person to be tested collected in real time by the nine-axis sensor, wherein the state includes: the non-exercise state, the first exercise state and the second exercise state, and the intensity of exercise of the second exercise state is higher than that of the first exercise state; the multi-light source heart rate measurement apparatus is applied to acquire a corresponding pulse signal according to the current exercise state of the person to be tested to determine a corresponding heart rate of the person to be tested based on the pulse signal. The acceleration data of the person to be tested collected by the nine-axis sensor in real time can judge the exercise state of the person to be tested more accurately to measure the heart rate corresponding to the exercise state.

In some embodiments of the present application, the applying the multi-light source heart rate measurement apparatus to acquire the corresponding pulse signal according to the current state of the person to be tested to determine the heart rate corresponding to the person to be tested based on the pulse signal, includes:
if the current state of the person to be tested is the non-exercise state, controlling the second light source 4 to emit any one of red light, infrared light and green light to a skin of the person to be tested currently in the non-exercise state, controlling the first optical receiving group 1 to correspondingly receive a pulse signal reflected by the skin, and determining a current resting heart rate of the person to be tested based on the pulse signal;
or, if the current state of the person to be tested is the non-exercise state, controlling the first light source 3 to emit any one of red light, infrared light, and green light to the skin of the person to be tested currently in the non-exercise state, so that an optical signal reflected by blood and tissues of the person to be tested is received by the first optical receiving group 1 and/or the second optical receiving group 2, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines the current resting heart rate corresponding to the person to be tested based on the pulse signal.

Specifically, if the current state of the person to be tested is the non-exercise state, the second light source 4 is controlled to emit any one of red light, infrared light, and green light to the skin of the person to be tested currently in the non-exercise state, and the first optical receiving group 1 is controlled to correspondingly receive the pulse signal reflected by the skin, and the current resting heart rate of the person to be tested is determined based on the pulse signal; or if the current state of the person to be tested is the non-exercise state, the first light source 3 is controlled to emit any one of red light, infrared light, and green light to the skin of the person to be tested currently in the non-exercise state, so that the optical signal reflected by the blood and tissues of the person to be tested is received by the first optical receiving group 1 and/or the second optical receiving group 2, and the optical signal is converted into the electrical signal to be sent to the analog-to-digital conversion chip; the analog-to-digital conversion chip converts the electrical signal into the digital signal and sends the digital signal to the processor, so that the processor determines the corresponding pulse signal based on the digital information, and then determines the current resting heart rate corresponding to the person to be tested based on the pulse signal. Through multi-light path heart rate measurement, the accuracy of heart rate measurement of the person to be tested in non-exercise state can be improved.

In some embodiments of the present application, the applying the multi-light source heart rate measurement apparatus to acquire the corresponding pulse signal according to the current state of the person to be tested to determine the heart rate corresponding to the person to be tested based on the pulse signal, includes:
if the current state of the person to be tested is the first exercise state, controlling the first light source 3 to emit green light to a skin of the person to be tested currently in the first exercise state, so that an optical signal reflected by blood and tissues of the person to be tested is received by the first optical receiving group 1 and/or the second optical receiving group 2, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines a current first heart rate corresponding to the person to be tested based on the pulse signal.

Specifically, if the current state of the person to be tested is the first exercise state, the first light source 3 is controlled to emit green light to the skin of the person to be tested currently in the first exercise state, so that the optical signal reflected by the blood and tissues of the person to be tested is received by the first optical receiving group 1 and/or the second optical receiving group 2, and the optical signal is converted into the electrical signal to be sent to the analog-to-digital conversion chip; the analog-to-digital conversion chip converts the electrical signal into the digital signal and sends the digital signal to the processor, so that the processor determines the corresponding pulse signal based on the digital information, and then determines the current first heart rate corresponding to the person to be tested based on the pulse signal. Through multi-light path heart rate measurement, the accuracy of heart rate measurement of the person to be tested in the first exercise state can be improved.

In some embodiments of the present application, the applying the multi-light source heart rate measurement apparatus to acquire the corresponding pulse signal according to the current state of the person to be tested to determine the heart rate corresponding to the person to be tested based on the pulse signal, includes:
if the current state of the person to be tested is the second exercise state, controlling the second light source 4 to emit green light to a skin of the person to be tested currently in the second exercise state, so that an optical signal reflected by blood and tissues of the person to be tested is received by the second optical receiving group 2, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines a current second heart rate corresponding to the person to be tested based on the pulse signal.

Specifically, if the current state of the person to be tested is the second exercise state, the second light source 4 is controlled to emit green light to the skin of the person to be tested currently in the second exercise state, so that the optical signal reflected by the blood and tissues of the person to be tested is received by the second optical receiving group 2, and the optical signal is converted into the electrical signal to be sent to the analog-to-digital conversion chip; the analog-to-digital conversion chip converts the electrical signal into the digital signal and sends the electrical signal to the processor, so that the processor determines the corresponding pulse signal based on the digital information, and then determines the current second dynamic heart rate corresponding to the person to be tested based on the pulse signal. Through multi-light path heart rate measurement, the accuracy of heart rate measurement of the person to be tested in the second exercise state can be improved.

The present application provides a multi-light source heart rate measurement apparatus, a method and a wearable device, wherein the multi-light source heart rate measurement apparatus includes: a first optical receiving group, a second optical receiving group, a first light source and a second light source. Distances between the first light source and the first optical receiving group and the second optical receiving group respectively are both a preset resting and first dynamic heart rate measurement interval; the distance between the second light source and the first optical receiving group is a preset resting heart rate measurement interval, and the resting heart rate measurement interval is less than or equal to the resting and first dynamic heart rate measurement interval; the distance between the second light source and the second optical receiving group is a preset second dynamic heart rate measurement interval, and the second dynamic heart rate measurement interval is larger than the resting and first dynamic heart rate measurement interval. According to the present application, heart rate measurement under multiple scenes of resting, slight exercise, and vigorous exercise can be achieved without using the accelerometer to participate in the calculation of PPG by setting the intervals between different light sources and different optical receiving groups.

Embodiments of the present application further provide an electronic device, such as a central server, and the electronic device may include a processor, a memory, a receiver, and a transmitter, the processor is configured to perform the multi-light source heart rate detection method described in the above embodiments, wherein the processor and the memory may be connected through a bus or other means, for example, by a bus connection. The receiver may be connected to the processor and memory by wired or wireless means.

The processor may be a Central Processing Unit (CPU). The processor may also be another general-purpose processors, a Digital Signal Processor (DSP), an Application Specific Integrated Circuit (ASIC), a Field-Programmable Gate Array (FPGA) or other programmable logic devices, a discrete gate or transistor logic device, a discrete hardware component, or a combination thereof.

Embodiments of the present application further provide a computer-readable storage medium having stored thereon a computer program, which, when executed by a processor, implements the steps of the multi-light source heart rate detection method in the preceding embodiments. The computer-readable storage medium may be a tangible storage medium such as a random-access memory (RAM), a memory, a read only memory (ROM), an electrically programmable ROM, an electrically erasable programmable ROM, a register, a floppy disk, a hard disk, a removable storage disk, a CD-ROM, or any other form of storage medium known in the art.

In the present application, features that are described and/or illustrated herein with respect to one embodiment may be used in the same way or in a similar way in one or more other embodiments and/or in combination with or substituted for features of other embodiments.

## Claims

1. A multi-light source heart rate measurement apparatus, **characterized by** comprising: a first optical receiving group, a second optical receiving group, a first light source and a second light source;
wherein distances between the first light source and the first optical receiving group and the second optical receiving group respectively are both a preset resting and first dynamic heart rate measurement interval, based on the first light source cooperating with the first optical receiving group and/or the second optical receiving group, a resting heart rate of a person to be tested in a non-exercise state or a first dynamic heart rate of the person to be tested in a first exercise state is measured;
the distance between the second light source and the first optical receiving group is a preset resting heart rate measurement interval, and the resting heart rate measurement interval is less than or equal to the resting and first dynamic heart rate measurement interval; the resting heart rate of the person to be tested is measured based on the second light source cooperating with the first optical receiving group;
the distance between the second light source and the second optical receiving group is a preset second dynamic heart rate measurement interval, and the second dynamic heart rate measurement interval is larger than the resting and first dynamic heart rate measurement interval, a second dynamic heart rate of the person to be tested in a second exercise state is measured based on the second light source cooperating with the second optical receiving group, wherein an intensity of exercise of the second exercise state is greater than that of the first exercise state.

2. The multi-light source heart rate measurement apparatus according to claim 1, **characterized in that** the first light source and the second light source are respectively in communication connection with a processor of a wearable device such that the processor controls at least one of the first light source and the second light source to be turned on or off.

3. The multi-light source heart rate measurement apparatus according to claim 1, **characterized in that** the first light source and the second light source are both light-emitting diodes for emitting red light, infrared light, and green light;
the first light source emits the green light when used to measure the first dynamic heart rate of the person to be tested in the first exercise state and the second light source emits the green light when used to measure the second dynamic heart rate of the person to be tested in the second exercise state.

4. The multi-light source heart rate measurement apparatus according to claim 1, **characterized in that** both the first optical receiving group and the second optical receiving group comprise at least two optical receivers;
the distance between each of the optical receivers and the first light source is the resting and first dynamic heart rate measurement interval;
the distances between the optical receivers in the first optical receiving group and the second light source are both the resting heart rate measurement interval; and
the distances between the optical receivers in the second optical receiving group and the second light source are both the second dynamic heart rate measurement interval.

5. The multi-light source heart rate measurement apparatus according to claim 1, **characterized in that** a value of the resting heart rate measurement interval ranges from 2 mm to 5 mm;
the value of the resting and first dynamic heart rate measurement interval ranges from 4 mm to 5 mm; and
the value of the second dynamic heart rate measurement interval ranges from 5 mm to 9 mm.

6. A wearable device, **characterized by** provided with a nine-axis sensor and the multi-light source heart rate measurement apparatus according to any one of claims 1 to 5;
the multi-light source heart rate measurement apparatus is arranged on a housing of the wearable device for contacting a skin of the person to be tested; the first optical receiving group, the second optical receiving group, the first light source and the second light source are respectively in communication connection with a processor in the wearable device;
the nine-axis sensor is used for monitoring an acceleration data of the person to be tested in real time, and sending the acceleration data to the processor in the wearable device in real time, so that the processor determines a current intensity of exercise of the person to be tested based on the acceleration data, and controls at least one of the first light source and the second light source to be turned on or off according to the intensity of exercise, so that an optical signal reflected by blood and tissues of the person to be tested is received by the first optical receiving group and/or the second optical receiving group, and the optical signal is converted into an electrical signal to be sent to an analog-to-digital conversion chip; the analog-to-digital conversion chip converts the electrical signal into a digital signal and sends the digital signal to the processor, so that the processor determines a corresponding pulse signal based on a digital information, and then determines a heart rate corresponding to the person to be tested based on the pulse signal.

7. A multi-light source heart rate detection method, **characterized by** implemented by using the multi-light source heart rate measurement apparatus according to any one of claims 1 to 5;
the multi-light source heart rate detection method comprises:
judging a current state of the person to be tested based on an acceleration data of the person to be tested collected in real time by a nine-axis sensor, wherein the state comprises: the non-exercise state, the first exercise state and the second exercise state, and the intensity of exercise of the second exercise state is higher than that of the first exercise state; and
applying the multi-light source heart rate measurement apparatus to acquire a corresponding pulse signal according to the current exercise state of the person to be tested to determine a heart rate corresponding to the person to be tested based on the pulse signal.

8. The multi-light source heart rate detection method according to claim 7, **characterized in that** the applying the multi-light source heart rate measurement apparatus to acquire the corresponding pulse signal according to the current state of the person to be tested to determine the heart rate corresponding to the person to be tested based on the pulse signal, comprises:
if the current state of the person to be tested is the non-exercise state, controlling the second light source to emit any one of red light, infrared light and green light to a skin of the person to be tested currently in the non-exercise state, controlling the first optical receiving group to correspondingly receive a pulse signal reflected by the skin, and determining a current resting heart rate of the person to be tested based on the pulse signal;
or, if the current state of the person to be tested is the non-exercise state, controlling the first light source to emit any one of red light, infrared light, and green light to the skin of the person to be tested currently in the non-exercise state, so that an optical signal reflected by blood and tissues of the person to be tested is received by the first optical receiving group and/or the second optical receiving group, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines the current resting heart rate corresponding to the person to be tested based on the pulse signal.

9. The multi-light source heart rate detection method according to claim 7, **characterized in that** the applying the multi-light source heart rate measurement apparatus to acquire the corresponding pulse signal according to the current state of the person to be tested to determine the heart rate corresponding to the person to be tested based on the pulse signal, comprises:
if the current state of the person to be tested is the first exercise state, controlling the first light source to emit green light to a skin of the person to be tested currently in the first exercise state, so that an optical signal reflected by blood and tissues of the person to be tested is received by the first optical receiving group and/or the second optical receiving group, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines a current first heart rate corresponding to the person to be tested based on the pulse signal.

10. The multi-light source heart rate detection method according to claim 7, **characterized in that** applying the multi-light source heart rate measurement apparatus to acquire the corresponding pulse signal according to the current state of the person to be tested to determine the corresponding heart rate of the person to be tested based on the pulse signal, comprises: if the current state of the person to be tested is the second exercise state, controlling the second light source to emit green light to a skin of the person to be tested currently in the first exercise state, so that an optical signal reflected by blood and tissues of the person to be tested is received by the second optical receiving group, and the optical signal is converted into an electrical signal to be sent to an analog-to-digital conversion chip; the analog-to-digital conversion chip converts the electrical signal into a digital signal and sends the digital signal to the processor, so that the processor determines the corresponding pulse signal based on a digital information, and then determines a current second heart rate corresponding to the person to be tested based on the pulse signal.
